# EUROPÄISCHE PATENTSCHRIFT

(11) **EP 2 407 216 B1**
(45) Veröffentlichungstag und Bekanntmachung des Hinweises auf die Patenterteilung: **24.09.2014**
(21) Anmeldenummer: 11000568.3
(22) Anmeldetag: 28.08.2008
(51) Int. Cl.: A63B 24/00, A63B 22/08, A61B 5/22, A63B 22/06

(54) **Ergometrisches Trainingsgerät**
Ergometric training device
Appareil d'entraînement ergométrique

(30) Priorität: 30.08.2007 AT 13632007; 30.08.2007 AT 13642007
(43) Veröffentlichungstag der Anmeldung: 18.01.2012
(62) Teilanmeldung aus: 08782834.9
(73) Patentinhaber: Wattbike IP Limited, Nottingham NG11 7HQ (GB)
(72) Erfinder: Bacanovic, Milan, 1210 Wien (AT); Adamovic, Dusan, 1230 Wien (AT)
(74) Vertreter: Potter Clarkson LLP

(56) Entgegenhaltungen:
- DE-A1- 4 227 586
- US-A- 4 934 692
- US-A- 5 027 303

## Beschreibung

Die Erfindung betrifft eine ergometrische stationäre Übungsvorrichtung mit einem manuell (von Hand oder Fuß) betriebenen Antrieb mit zwei wechselweise bedienbaren Antriebselementen, wobei der Antrieb über ein Getriebe mit einem Schwungrad verbunden ist, sowie mit einem Messmittel zur Messung der über den Antrieb aufgebrachten Antriebskraft bzw. dieser zugeordneten Drehmomentkraft und einer Messanordnung zur Messung der Bewegungsposition, insbesondere Winkelposition, des Antriebs. Die Antriebselemente sind vorzugsweise Pedale, ähnlich jenen eines Fahrrads, können jedoch auch anderer Art sein wie z.B. die Trittfelder eines sogenannten Steppers.

Eine Trainingsvorrichtung dieser Art ist in US 5,027,303 beschrieben. Zur Messung von Parametern wie Drehmoment, Arbeit, Leistung, Winkelgeschwindigkeit und Dauer einer Umdrehung an einer Pedal-Anordnung wird das Drehmoment mittels Dehnmessstreifen gemessen, die an belasteten Komponenten der Pedal-Anordnung angebracht sind. Dadurch erfolgt eine Messung des gesamten Drehmoments sowie der Drehmomente am linken und rechten Pedal (linkes bzw. rechtes Bein); daraus kann die verrichtete Arbeit und die Leistung berechnet werden.

EP 0 925 096 B1 beschreibt ein elektronisches Übungssystem mit einem Monitor für physische Aktivität, der eine Sensor- und Aufzeichnungs-Vorrichtung aufweist, welche während einer ersten Periode physischer Aktivität Daten erfasst und aufzeichnet. Die Übungsvorrichtung weist einen Widerstandserzeuger, z.B. eine Wirbelstrombremse, und eine Steuerung auf, die die aufgezeichneten Daten der physischen Aktivitäten verwendet, um den Betrieb der Übungsvorrichtung zu steuern.

US 5,354,251 beschreibt eine Übungsmaschine, bei der auf einem lang gestreckten Rahmen ein Sitz und einer federbelasteten Drehwelle befestigt sind. Die Drehwelle ist mit einem Schwungrad verbunden und weist Widerstandsgeräte auf. Als Widerstandsgeräte sind beispielsweise eine Zentrirugalbremse, ein Windrad-artig durchbrochenes Schwungrad, sowie ein Wirbelstrom-Bremsrad, in das ein Windrad zur Kühlung integriert ist, offenbart.

Weitere Übungsgeräte sind in US 2002/0004439 A1, US 2007/0117680 A1, US 5,611,759 und US 5,749,807 beschrieben.

Aus der JP 05 201374 A geht eine Drehmomentmessung an der Kette eines Fahrrads hervor. Am oberen Kettenstrangteil ist ein Spannungsdetektor zur Messung der Spannkraft angeordnet, nämlich ein Zahnrad, das die Kette von außen berührt, und ein Dehnmessstreifen, der die von der Kette auf das Zahnrad ausgeübte Kraft misst.

DE 19919154 A1 beschreibt ein Verfahren und eine Vorrichtung zum Aufbringen einer Vorspannkraft auf ein Endlos-Treibelement, insbesondere eine Kette. Eine Andrückschiene wird mittels eines Kettenspanners mit einer vorbestimmten Kraft von außen auf die Kette aufgedrückt. Die so entstehende Vorspannkraft an der Kette wird über eine Steuerelektronik in Abhängigkeit von Sensordaten betreffend Schwingungsdaten oder anderen maßgebenden Kenngrößen eingestellt.

US 4,141,245 beschreibt ein Gerät zur Messung mechanischer Arbeit und Leistung, die an einem Treibelement zwischen zwei Antriebsrädern übertragen wird. Ein Kraftmesselement mit einer Rolle wird mittels Federkraft gegen das Treibelement gedrückt, und das Ausmaß der Auslenkung dient der Messung der übertragenen Zugkraft. Verschiedene Ausführungsformen enthalten eine innen oder außen anliegende Rolle, oder eine Kombination von zumindest einer innen und zumindest einer außen anliegenden Rolle.

Weitere Messeinrichtungen zum Messen des Antriebsdrehmoments bei einem Antriebssystem, z.B. eines Fahrrads, sind in US 4,909,086 und US 2007/0099735 A1 vorgestellt.

DE 42 27 586 A1 zeigt eine Pedal-Übungsvorrichtung mit getrennter Kraftmessung für beide Pedalhebel, nämlich mittels je eines Dehnmessstreifens an den beiden Pedalhebeln, und mit einem Winkelgeber, wodurch eine Auswertung des Bewegungsablaufs z.B. als Polardiagramm ermöglicht wird. DE 44 35 174 A1 schlägt zudem vor, die Dehnmessstreifen schräg auf den jeweiligen Pedalhebel anzuordnen.

Weitere Ansätze zur Kraftmessung im Verlauf der Pedalbewegung sind in US 2007/0149364 A1, US 5,573,481, WO 02/47551 A2 und EP 1362 552 A1 beschrieben.

Diese bekannten Übungs- und Messeinrichtungen erzielen die Messung der von dem Trainierenden aufgewendeten Kraft bzw. des zugeordneten Drehmoments auf verschiedene Wege, die jedoch häufig sehr aufwendig und komplex sind. Besonders dann, wenn eine differenzierte Betrachtung verschiedener Abschnitte des Ablaufs, nämlich eine Aufteilung zwischen den beiden Füßen (oder bei handbetätigten Geräten zwischen den beiden Händen) erwünscht ist, sind die bekannten Methoden sehr aufwändig.

Es ist eine Aufgabe der vorliegenden Erfindung, eine Übungsvorrichtung zu schaffen, bei der eine Messung der aufgebrachten Kraft/Drehmomentkraft aufgeteilt auf die links/rechts erfolgenden Bewegungen möglich ist.

Diese Aufgabe wird ausgehend von einer Übungsvorrichtung der eingangs genannten Art gelöst, bei welcher erfindungsgemäß die Vorrichtung ein Paar von Sensormitteln aufweist, die in Bezug auf ein mit dem Antrieb bewegungssynchron verbundenes Rad an Positionen relativ zu dem Rad angeordnet sind, wodurch ein Signal erzeugt wird, wenn das Rad an einer von zwei spezifischen Winkelpositionen angeordnet ist, wobei die spezifischen Winkelpositionen um 180° versetzt sind und Bewegungspositionen eines Lastwechsels zwischen den wechselweise bedienbaren Antriebselementen entsprechen.

Diese Lösung gestattet auf einfache Weise eine Detektion der Lastwechsel zwischen der linken und der rechten Extremität, und somit eine Unterscheidung zwischen den von diesen aufgewendeten Kräften bzw. erbrachten Arbeit. Zudem ergibt dies eine Vereinfachung des Messvorgangs sowie eine zuverlässigere Auswertung der aufgenommenen Daten der kraft als Funktion der Fußposition bzw. des Drehwinkels. Das Rad ist z.B. ein auf der Pedalwelle drehfest angebrachtes Zahnrad, oder kann mit der Pedalwelle über ein Getriebe verbunden sein, sofern die Umsetzung einen hinreichenden Rückschluss von der Winkelstellung des Rades auf die Bewegungsstellung des Antriebs ermöglicht.

In einer bevorzugten Ausführungsform der Erfindung, die eine besonders effektive Realisierung des der Erfindung zugrunde liegenden Lösungswege darstellt, sind die beiden Sensormittel als auf dem Rad an zueinander gegenüber liegenden Positionen befestigten Sensorstücke ausgebildet; zudem ist zumindest ein Sensor ortsfest angeordnet, mithilfe dessen das Passieren der Sensorstücke bei einer spezifischen Winkelposition des Rades detektierbar ist, wobei die Winkelposition einer Bewegungsposition eines Lastwechsels zwischen den beiden Antriebselementen entspricht.

Es ist jedoch ebenfalls zweckmäßig, wenn die beiden Sensormittel als ortsfest angeordnete Sensoren ausgebildet sind, und zudem zumindest ein auf dem Rad befestigtes Sensorstück vorgesehen ist, wobei mithilfe der Sensoren das Passieren des zumindest einen Sensorstücks an spezifischen, zueinander gegenüber liegenden Winkelpositionen des Rades detektierbar ist, wobei die Winkelpositionen jeweils einer Bewegungsposition eines Lastwechsels zwischen den beiden Antriebselementen entsprechen.

Für eine effektive berührungslose Detektion der beweglichen Teile ist es günstig, wenn die Sensorstücke Magnete, insbesondere Permanentmagnete, sind und die Sensoren Magnetfeld-Sensoren sind.

Um darüber hinaus eine Vereinfachung der zur Messung der aufgebrachten Kraft verwendeten Messeinrichtungen zu erreichen, ist es vorteilhaft, wenn das Messmittel zur Messung der Antriebskraft ein an ein Zugmittel, insbesondere an eine Kette, des Getriebes angelegter Arm ist, der das Zugmittel seitlich geringfügig eindrückt und einen Messsensor zum Messen der dabei von dem Zugmittel ausgeübten Stellkraft aufweist.

Vorteilhafter Weise kann eine Auswerteeinrichtung vorgesehen sein, um Signale von dem Messmittel betreffend die aufgebrachte Antriebskraft bzw. dieser zugeordnete Drehmomentkraft entgegenzunehmen und aufgrund der von dem Messmittel gelieferten Signale den zeitlichen Verlauf der Antriebskraft bzw. der Drehmomentkraft sowie davon ableitbaren Größen zu berechnen und laufend auszugeben. Die Auswerteeinrichtung kann außerdem Signale von der Messanordnung betreffend Zeiten der Lastwechsel entgegennehmen und die berechneten Größen in Abhängigkeit von der von der Messanordnung gemeldeten Lastwechsel wechselweise einer rechten oder linken Extremität eines Trainierenden zuordnen. Die Ausgabe der so berechneten Größen kann somit aufgrund des Signals der Messanordnung betreffend Zeiten der Lastwechsel aufgeteilt auf die rechte bzw. linke Extremität erfolgen. Durch diese Weiterbildung gelingt eine unkomplizierte Bestimmung und automatisierte Ausgabe der nach Links/Rechts aufgeteilten Trainingsleistung.

Des Weiteren ist es wünschenswert, dass der geschwindigkeitsabhängige Widerstand, den der Trainierende auf dem erfindungsgemäßen Übungsgerät überwinden muss, möglichst naturgetreu ist, d.h. dem Widerstand auf einem straßentauglichen Fahrrad entspricht. Zu diesem Zweck ist es günstig, wenn das Schwungrad eine durch Luftreibung gebremste Vorrichtung aufweist und mit einer elektromagnetisch wirkenden Bremse verbunden ist. Die durch Luftreibung gebremste Vorrichtung kann ein mit dem Schwungrad drehfest verbundenes Schaufelrad sein. Zudem kann das Schaufelrad eine Vielzahl von parallel zur Drehachse ausgerichteten Schaufelflächen aufweisen.

Um darüber hinaus die Luftreibungswirkung nach Bedarf einstellen zu können, ist es vorteilhaft, wenn die durch Luftreibung gebremste Vorrichtung sich in einem Gehäuse befindet, das Mittel zur Einstellung der Menge des infolge der Bewegung des Schwungrads bewegten Luftstroms aufweist. Beispielsweise kann das Gehäuse Öffnungen aufweisen, deren Weite und/oder Luftdurchlässigkeit verstellbar ist und mit deren Hilfe der durch das Gehäuse gehend Luftstrom einstellbar ist.

Die Erfindung wird nachstehend anhand eines nicht einschränkenden Ausführungsbeispiels näher erläutert, das in den beigefügten Zeichnungen dargestellt ist. Die Zeichnungen zeigen:
- Fig.1: eine perspektivische Ansicht (von rechts vorne) des Trainingsgeräts nach dem Ausführungsbeispiel der Erfindung;
- Fig. 2 und 3: das Trainingsgerät in einer weiteren Schrägansicht und einer Seitenansicht von der linken Seite;
- Fig. 4: das Getriebe des Trainingsgeräts in einer Detailansicht (Seitenansicht von rechts ohne Gehäuse);
- Fig. 5: ein Detail der Fig. 4 mit der Kraftmessung an der Kette des Getriebes;
- Fig. 6: eine Schnittansicht der Radtrommel des Trainingsgeräts;
- Fig. 7: eine Ansicht des Trainingsgeräts mit eröffneter Magnetbremse;
- Fig. 8 und 9: Detailansichten von links auf den Bereich der Pedalanordnung, mit teilweise entferntem Gehäuse, sodass die Sensoren zur Messung der Pedalstellung sichtbar sind, wobei in Fig. 7 außerdem der Trägerholm und das Achslager fortgelassen sind;
- Fig. 10: ein Blockdiagramm der Signal- und Datenauswertung;
- Fig.11: eine Aufsicht auf das Griffteil des Trainingsgeräts mit einer Anzeige, und
- Fig. 12: ein Beispiel einer Darstellung der Antriebskraft in Abhängigkeit von dem Drehwinkel (Polarform).

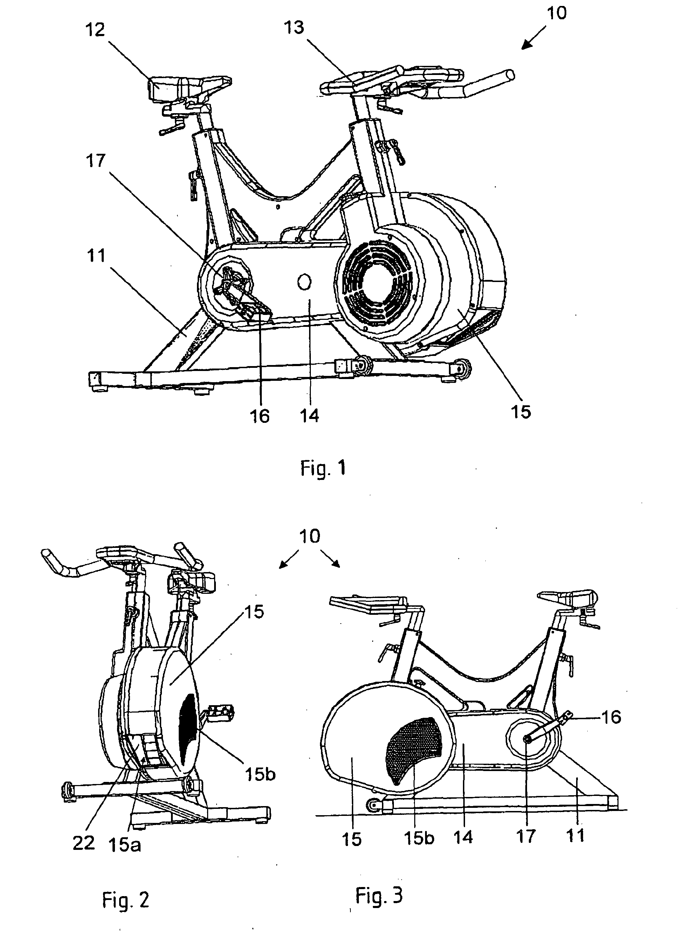

Das im Folgenden eingehend behandelte Ausführungsbeispiel betrifft ein stationäres ergometrisches Fahrrad-Trainingsgerät, das in Fig. 1 bis 3 in verschiedenen Ansichten gezeigt ist. Das Trainingsgerät 10 kann beispielsweise als Heimtrainer, als Trainingsgerät in einem Fitness-Studio oder zur Verwendung im Spitzensport, oder auch im medizinischen Bereich verwendet werden.

Das Trainingsgerät 10 weist einen fahrradartigen Gestellrahmen 11 mit Sitz 12 und Griffteil 13 auf, deren Position jeweils einstellbar ist, während eines Trainingsablaufs jedoch fest ist. Im Fußbereich befindet sich ein Gehäuse 14, das im vorderen Bereich einen Radkasten 15 aufweist, sowie ein Paar Pedale 16. Die Pedale 16 sind nach bekannter Art an einer Pedalwelle 17 befestigt und stehen über ein Getriebe mit Widerstandsmitteln in Verbindung, die wie nachstehend beschrieben in dem Radkasten 15 untergebracht sind.

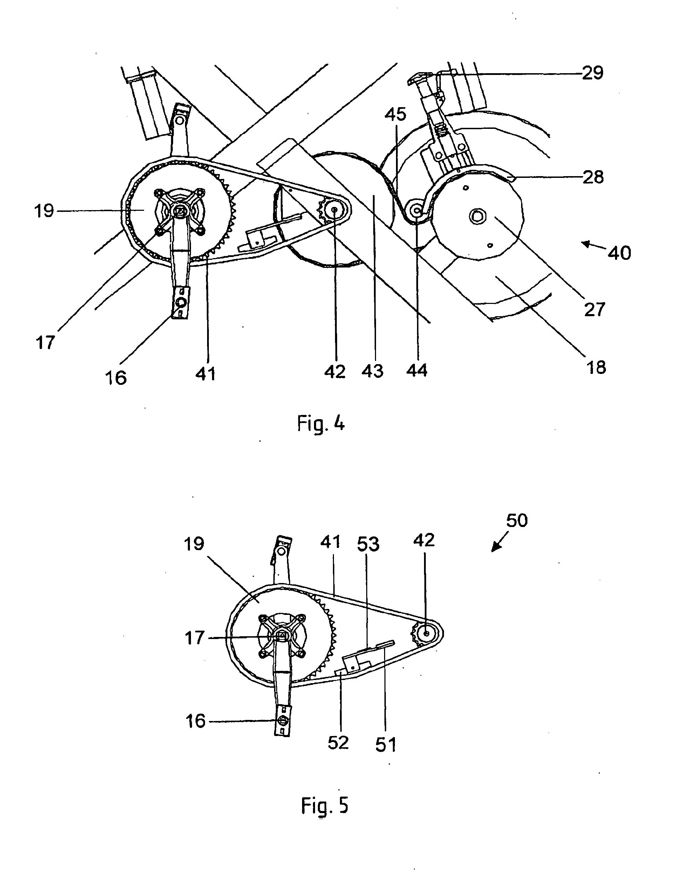

Bezugnehmend auf Fig. 4 ist das Getriebe 40 im gezeigten Ausführungsbeispiel eine Kombination zweier Zugmittelgetriebe, nämlich eines Zahnradgetriebes mit einem Riemengetriebe, wodurch eine hohe Übersetzung der Bewegung vom Pedal 16 auf das Schwungrad 18 erreicht wird. Die Pedale 16 sind über die Pedalwelle 17 starr mit einem Zahnrad 19 verbunden, das über eine Kette 41 ein Ritzelrad 42 antreibt. Das Ritzelrad 42 wiederum ist mit einem Scheibenrad 43 verbunden, das über einen mittels eines Hilfsrads 44 gespannten Riemen 45 das Schwungrad 18 antreibt.

Die gezeigte Ausführungsform verfügt über ein Messsystem mit einer Messgenauigkeit von 2% oder besser. Es dient zur Messung der vom Benutzer aufgewendeten Kraft und der Pedalgeschwindigkeit und ist mit einem Computersystem zur Darstellung und Auswertung der Messdaten verbunden.

### Kraftmessung

Wie in Fig. 5 gezeigt, ist vorzugsweise im ersten Zugmittelgetriebe vorgesehen ein Messmittel 50 zur Messung der Kraft vorgesehen, die der Trainierende über den Pedalantrieb auf die Kette 41 aufbringt. Da die Pedallänge fest und bekannt ist, ist die Antriebskraft in das wirkende Drehmoment ("Drehrnomentkraft") direkt umzurechnen und insofern äquivalent.

Das Messmittel ist vorzugsweise als Biegebalken mit einem Messdehnstreifen realisiert, der die Kette geringfügig auslenkt und die Rückstellkraft misst. Ein am Rahmen 11 befestigter Arm 51 trägt an seinem Ende ein Gleitstück 52, das beispielsweise aus Kunststoff gefertigt ist. Das Gleitstück ist an die Kette 41 beispielsweise von innen angelegt, ähnlich einem Kettenspanner, und drückt die Kette geringfügig nach außen. Wenn die Kette infolge einer vom Trainierenden aufgebrachten Kraft unter Spannung steht, ergibt sich eine tangentiale Komponente der Kraft auf das Kunststoffgleitstück und auf das Gleitstück wirkt eine rückstellende Kraft, die proportional der Kettenspannung und somit der Drehmomentkraft ist. Die dadurch sich ergebende elastische Biegung des Armes 51 wird von einem Messsensor, z.B. einem Dehnungsmessstreifen 53, gemessen. Das Signal des Messsensors wird elektronisch ausgewertet, wie weiter unten erläutert ist.

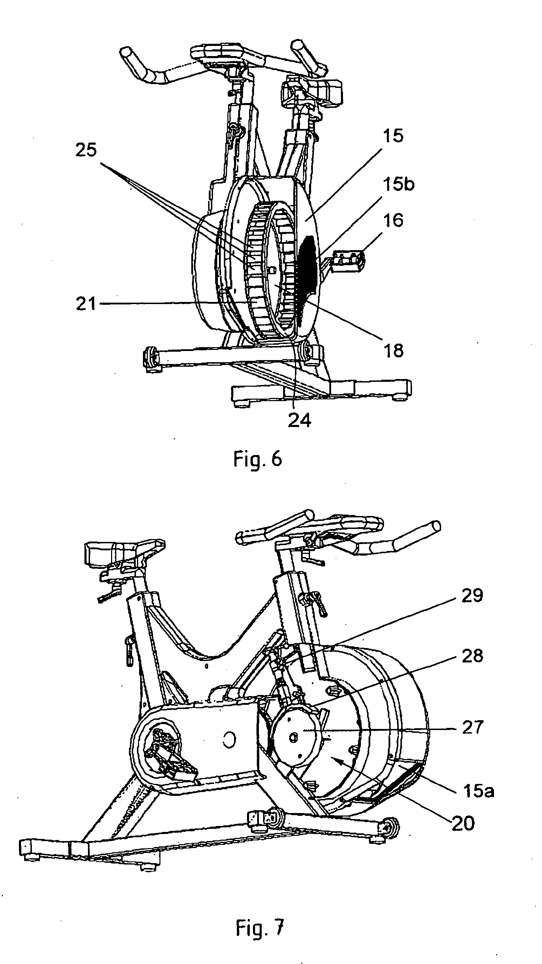

Zur Kalibrierung der Kraftmessung wird beispielsweise ein Gewicht bekannter Größe an einem der Pedale 16 befestigt, und die Drehung wird an dem Schwungrad 18 bzw. der Schwungscheibe 27 (Fig. 7) mithilfe eines Blockiermittels (nicht gezeigt) mechanisch blockiert. Die in diesem Zustand gemessene Kraft dient durch Vergleich mit der bekannten, durch das Gewicht aufgeprägten Kraft als Grundlage für die Kalibrierung des Kraftmesssystems.

### Widerstandsmittel

Bezugnehmend auf Fig. 6 weist das durch die Pedalbewegung über das Getriebe 60 angetriebene Schwungrad 18 ein Luftschaufelrad 21 auf, das in der gezeigten Ausführungsform drehfest dem Umfang des Schwungradblattes aufgesetzt ist. Das Luftschaufelrad 21 befindet sich in einem eigenen Behälter als Teil des Radkastens 15.

Wie in Fig. 7 ersichtlich ist, ist im gezeigten Ausführungsbeispiel eine Wirbelstrombremse 20 auf derselben Achse wie das Schwungrad 18, vorzugsweise dieser gegenüber liegend, angeordnet. Die Wirbelstrombremse 20 ist beispielsweise eine Magnetbremse, bei der eine metallische Schwungscheibe 27 mit stellbaren (Permanent-)Magneten 28 nach bekannter Art zusammenwirkt; alternativ kann auch eine andere elektromagnetisch wirkende Bremse realisiert sein. In dem gezeigten Ausführungsbeispiel sind die Magnete auf einem Stahlbügel entlang eines Umfangsstücks der Scheibe 27 angeordnet und werden mithilfe eines Stellmechanismus 29 zu der Scheibe 27 hin bzw. von dieser weg positioniert. Die Scheibe 27 besteht beispielsweise aus Stahl, der mit einem Kupferring ummantelt ist. Um die Drehung gänzlich blockieren zu können, sind beispielsweise in der Scheibe 27 zwei Löcher vorgesehen, in die ein (nicht gezeigter) im Gehäuse oder am Rahmen gehaltener Blockierstift von der Seite eingeführt werden kann.

Die Widerstandmittel der erfindungsgemäßen Trainingsvorrichtung wurden denen nachgebildet, die bei einer Radfahrt auftreten. Die beim Radfahren wirkenden Widerstände sind (a) Luftwiderstand, (b) Reibung der mechanischen Teile innerhalb des Fahrrads und (c) Rollwiderstand zwischen Reifen und der Oberfläche der Fahrbahn oder Steigung des Geländes. Der Luftwiderstand macht in der Regel den überwiegenden Teil - häufig mehr als 90% - des Gesamtwiderstands aus und wächst quadratisch mit der Geschwindigkeit. Somit wächst die erbrachte Leistung mit der dritten Potenz der Geschwindigkeit. Die Reibung im Fahrrad und der Rollwiderstand wachsen linear mit der Geschwindigkeit, was einer Leistung mit quadratischer Geschwindigkeitsabhängigkeit entspricht.

Bei dem Trainingsgerät 10 wird zur Simulierung dieser beiden Widerstandsarten ein kombiniertes Bremssystem verwendet, welches zwei Bremsteilsysteme aufweist, nämlich wie bereits beschrieben ein über Luftbremsung wirkendes Bremsmittel in Form des Rads 21 und eine elektromagnetisch wirkende Bremse 20. Auf diese Weise gelingt eine realistische Modellierung des Widerstandsverhaltens eines Fahrrads, was das Gefühl vermittelt, sich auf einem "normalen' Fahrrad zu bewegen. Die beiden Teilsysteme können unabhängig voneinander eingestellt werden. Sie haben keinen Einfluss auf die weiter unten beschriebenen Messvorrichtungen. Die Kombination der beiden Bremsteilsysteme ermöglicht eine große Widerstandsspanne, die sich in Abhängigkeit von der Trittfrequenz ergibt. Es sind keine externen Energiequellen notwendig.

Wieder bezugnehmend auf Fig. 6 hat das Luftschaufelrad 21 eine im Wesentlichen Zylinderring-artige Gestalt. Entlang des Umfangs ist zwischen zwei seitlichen Halteringen 24 in regelmäßigen Abständen eine Anzahl von Schaufelflächen 25 angeordnet, die jeweils blattartig parallel zur Drehachse des Rads 21 und in einem von 90° verschiedenen Winkel zum Radius ausgerichtet sind. Wenn das Rad 21 sich dreht, so bewegen die Schaufelflächen 25 die umgebende Luft nach innen. Auf diese Weise wird Luft durch das Seitenfenster 15b angesaugt und über die an der unteren Vorderseite des Radkastens 15 befindliche Öffnung 15a (Fig. 2) wieder hinausgedrückt; somit wird das Rad 21 durch die sich ergebende Luftumwälzung gebremst.

Im Gegensatz zu bekannten Trainingsvorrichtungen mit einer Luftradbremse kann der Widerstand bei der gezeigten Vorrichtung durch Regulierung der Luftzufuhr auf der Statorseite eingestellt werden (Fig. 3), nämlich durch mehr oder weniger weitgehendes Schließen der Öffnung 15a mittels einer Klappe 22 und/oder Einstellen des Seitenfensters 15b hinsichtlich seiner Luftdurchlässigkeit, beispielsweise nach Art einer Jalousie. Dadurch kann die vom Luftwiderstand herrührende Bremswirkung in einem weiten Bereich eingestellt werden. Insbesondere kann durch Schließen der Öffnung 15a und des Fensters 15b der Widerstand auf Minimalwert nahe Null gestellt werden, sodass im Wesentlichen lediglich die mechanische Reibung im System verbleibt.

Durch diese Maßnahmen kann der Widerstand jeweils für beide Bremsteilsysteme eingestellt werden. Bei der gezeigten Ausführungsform kann eine Widerstandswirkung von 0 bis zu 5000 W gewählt werden.

### Messung der Pedalgeschwindigkeit

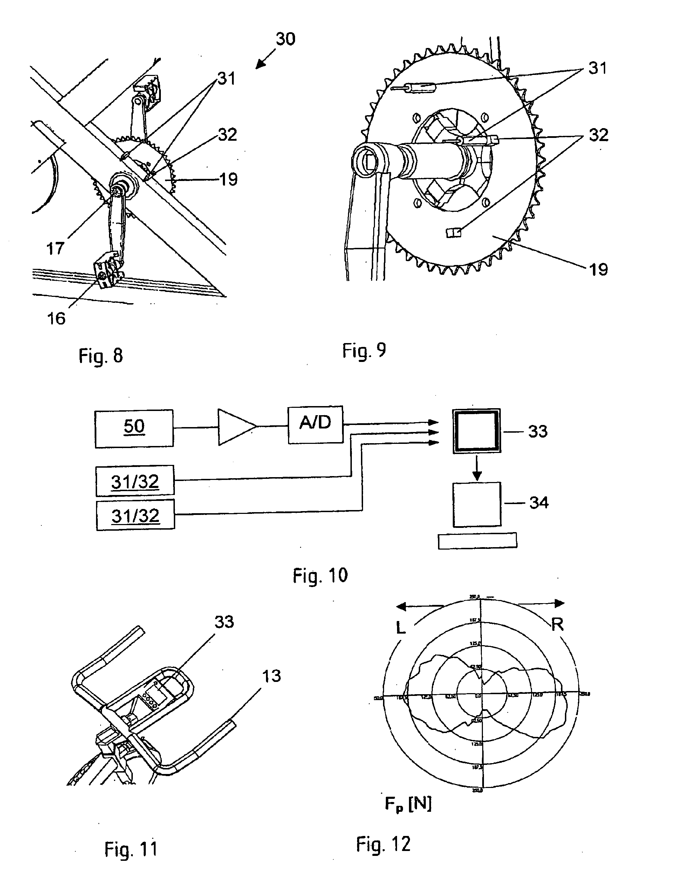

In Fig. 8 und 9 ist die Sensoranordnung 30 zur Messung der Pedalgeschwindigkeit dargestellt. Zwei Magnetfeld-Sensoren 31, z.B. Reed-Schalter, sind ortsfest neben dem Pedal-Zahnrad 19 angeordnet. Auf dem Zahnrad 19 befinden sich zwei Permanentrnagnete 32 an genau gegenüber liegenden Positionen, sodass jeder Magnet 32 im Laufe einer Drehung des Zahnrads 19 je einmal die Sensoren 31 passiert und so z.B. einen Signalimpuls erzeugt. Das sich so ergebende Signal wird einer Auswertung zugeführt und gestattet die genaue Messung der Drehzahl sowie - über die Kurbellänge der Pedale - der Pedalgeschwindigkeit. Die beiden Sensoren 31 und die zugehörenden Magnete 32 entsprechend einander paarweise und sind jeweils (um eine gegenseitige Auslösung des einen Sensors durch den Magneten des anderen Sensors auszuschließen) in verschiedenen Radialabständen zur Achse positioniert. Die Magnete sind hinsichtlich ihrer Winkellage zu den jeweils zugehörenden Sensoren in Bezug auf die Stellung der Pedale 16 so angeordnet, dass ein Signalimpuls eines Sensors 31 jeweils dann gegeben wird, wenn der Kraftwechsel von dem linken auf den rechten Pedal bzw. umgekehrt erfolgt. Wie in Fig. 8 und 9 erkennbar, ist bei der gezeigten Stellung mit 0° (rechtes Pedal senkrecht nach oben) der eine Magnet gerade auf Position mit dem ihm zugeordneten Sensor, während der andere Magnet sich genau gegenüber dem ihm zugeordneten Sensor befindet. Dies gestattet eine Aufteilung der Messung und getrennte Zuordnung zu dem linken bzw. rechten Fuß, und eine Rechts/ Links-Auswertung der von jeweils von einem Fuß erbrachten Kraft und Leistung, sowie einen Vergleich der beiden Fußleistungen (Balance).

Durch die Anordnung der Sensor-Magnet-Paare, sodass sie zur Detektion der Positionen der Lastwechsel eingerichtet sind, kann der Beginn der Messdurchläufe festgelegt werden, der üblicherweise aus einer Abfolge diskreter Messpunkte besteht. Ein Pedaldurchlauf an der Sensorposition - somit bei einem Lastwechsel - wird als Beginn einer Messreihe gewählt, sodass zum einen ein Messpunkt stets bei einem Lastwechsel erfolgen kann (wo insbesondere bei ungeübten Radfahrern ein Minimum der ausgeübten Kraft zu erwarten ist) und zum anderen die Messreihe zwischen aufeinanderfolgenden Sensordurchläufen bei im Wesentlichen gleichmäßiger Geschwindigkeit gemessen wird; denn nach einem Lastwechsel ist erfahrungsgemäß die Winkelgeschwindigkeit der Pedalbewegung im Wesentlichen konstant, wogegen oftmals die Geschwindigkeit zwischen den einzelnen Tretvorgängen wechseln kann. Dies ergibt eine Vereinfachung des Messvorgangs sowie eine zuverlässigere Auswertung der aufgenommenen Daten der Kraft als Funktion der Fußposition bzw. des Drehwinkels.

### Auswertung

Wie in Fig. 10 dargestellt, werden die von dem Kraftmesssensor (Dehnmessstreifen) 53 und den der Pedalmessung zugeordneten Sensoren 31 gelieferten Sensorsignale verstärkt, mittels Analog-Digital-Wandlern digitalisiert und einer elektronischen Auswertung, z.B. einer auf dem Griffteil befindlichen Trainingsanzeige 33 (Fig. 11) und/oder einem zugeordneten Computersystem 34, zugeführt. Auf dem Computersystem 34 werden die Signale in einen zeitabhängigen Verlauf der auf die Pedale aufgebrachten Antriebskraft umgerechnet, beispielsweise mit einer Datenrate von 100 Datenpunkten pro Sekunde, zudem können die Signale in Echtzeit dargestellt und/oder gespeichert werden. Die Daten können dann zu einem späteren Zeitpunkt abgerufen und bearbeitet werden. Die Darstellung der Daten erfolgt vorteilhafter Weise in einer auf die Pedalumdrehung bezogenen Weise und/oder in einer Polardarstellung wie in Fig.12 gezeigt.

Fig.12 zeigt ein Beispiel einer gemessenen Pedalkraft F_{P} (in N; der äußere Kreis entspricht 250 N) über eine ganze Umdrehung mit dem Pedal, als Funktion des Drehwinkels in einem Polardiagramm. Die gezeigten Winkel entsprechen direkt dem Winkel des Pedals, das im Uhrzeigersinn bewegt wurde, wobei 0° einer Stellung des rechten Pedals senkrecht nach oben entspricht. Es ist im Übrigen erwähnenswert, dass es bei der Antriebsbewegung insbesondere bei trainierten Sportlern zu einer Synergie zwischen den beiden Füßen kommt, und je besser die koordinative Fähigkeit beim Trainierenden ausgeprägt ist, desto runder ist die Figur der Kurve F_{P}.

Auf dem Computersystem 40 wird mittels geeigneter ergometrischer Software die Analyse der gemessenen Daten und graphische Darstellung am Bildschirm durchgeführt, beispielsweise:
■ Berechnung und Darstellung des Pedaldrehmoments,
■ Kraft als Funktion der Fußposition,
■ Drehzahl,
■ Geschwindigkeit (umgerechnet auf eine fiktive Fahrradgeschwindigkeit),
■ Leistung (W),
■ Durchschnittsleistung,
■ Energie (kJ, durch Integration),
■ Balance zwischen linkem und rechtem Fuß (in %),
■ Herzfrequenz (über zusätzlichen Sensorgurt, den der Benutzer trägt),
■ statistische Analysen.

Selbstverständlich ist die Erfindung nicht auf das beschriebene Ausführungsbeispiel beschränkt, sondern erstreckt sich auf alle Ausführungen, die unter den Bereich der Ansprüche fallen. Insbesondere kann die erfindungsgemäße Übungsvorrichtung auch andere Antriebselemente als Pedale aufweisen, beispielsweise Trittfelder wie bei einem Stepper oder ein Paar Handgriffe, die abwechselnd zu betätigen sind. Hierbei wird die Bewegung nach bekannter Art mechanisch über ein Getriebe in eine Drehbewegung eines Antriebsrades umgewandelt.

## Patentansprüche

1. Stationäre ergometrische Übungsvorrichtung mit einem hand- oder fußbetriebenen Antrieb mit zwei wechselweise bedienbaren Antriebselementen (16), vorzugsweise fußbetriebenen Pedalen, wobei der Antrieb über ein Getriebe mit einem Schwungrad (18) verbunden ist, sowie mit einem Messmittel (50) zur Messung der über den Antrieb aufgebrachten Antriebskraft bzw. dieser zugeordneten Drehmomentkraft und einer Messanordnung (30) zur Messung der Bewegungsposition, insbesondere der Winkelposition, des Antriebs,
**dadurch gekennzeichnet, dass** die Vorrichtung ein Paar von Sensormitteln (31, 32) aufweist, die in Bezug auf ein mit dem Antrieb bewegungssynchron verbundenes Rad (19) an Positionen relativ zu dem Rad (19) angeordnet sind, wodurch ein Signal erzeugt wird, wenn das Rad (19) an einer von zwei spezifischen Winkelpositionen angeordnet ist, wobei die spezifischen Winkelpositionen um 180° versetzt sind und Bewegungspositionen eines Lastwechsels zwischen den wechselweise bedienbaren Antriebselementen (16) entsprechen.

2. Übungsvorrichtung nach Anspruch 1, **dadurch gekennzeichnet, dass** die Messanordnung (30) ein Paar von auf dem Rad (19) befestigten Sensorstücken (32) und mindestens einen Sensor (31) aufweist, wobei der Sensor (31) ortsfest relativ zu dem Rad (19) angeordnet ist, wobei das Passieren eines Sensors (31) durch jedes Sensorstück (32) mittels der Sensorstücke (32) detektiert wird, die mit dem Rad (19) relativ zu dem mindestens einen Sensor (31) beim Betrieb des Antriebs bewegbar sind, wenn das Rad (19) in einer entsprechenden von zwei spezifischen Winkelpositionen angeordnet ist, wobei die Bewegungspositionen um 180° versetzt sind und den Bewegungsposition eines Lastwechsels zwischen den wechselweise bedienbaren Antriebselementen (16) entsprechen.

3. Übungsvorrichtung nach Anspruch 1, **dadurch gekennzeichnet, dass** die Messanordnung (30) ein ortsfest relativ zu dem Rad (19) angeordnetes Paar von Sensoren (31) und mindestens ein an dem Rad (19) befestigtes Sensorstück (32) aufweist, wobei das mindestens eine Sensorstück (32) mit dem Rad (19) relativ zu den Sensoren (31) beim Betrieb des Antriebs bewegbar ist, wodurch jeder der Sensoren (31) ein dieses passierendes Sensorstück (32) detektiert, wenn sich das Rad (19) in einer entsprechenden von zwei spezifischen Winkelpositionen in Bewegung befindet, wobei die Bewegungspositionen um 180° versetzt sind und den Bewegungsposition eines Lastwechsels zwischen den wechselweise bedienbaren Antriebselementen (16) entsprechen.

4. Übungsvorrichtung nach Anspruch 2 oder 3, **dadurch gekennzeichnet, dass** die Sensorstücke (32) Magnete und die Sensoren (31) Magnetfeldsensoren sind.

5. Übungsvorrichtung nach einem der vorangehenden Ansprüche, **dadurch gekennzeichnet, dass** das Messmittel (50) zur Messung der Antriebskraft ein an ein Zugmittel, insbesondere eine Kette, des Getriebes angelegter Arm ist, der das Zugmittel geringfügig eindrückt, und dass das Messmittel (50) weiterhin einen Messsensor (53) zum Messen der dabei von dem Zugmittel auf den Arm ausgeübten Stellkraft aufweist.

6. Übungsvorrichtung nach einem der vorhergehenden Ansprüche, **gekennzeichnet durch** eine Auswerteeinrichtung (40, 41), die dazu eingerichtet ist, Signale von dem Messmittel (50) betreffend die aufgebrachte Antriebskraft bzw. die dieser zugeordnete Drehmomentkraft entgegenzunehmen und aufgrund der von dem Messmittel (50) gelieferten Signale den zeitlichen Verlauf der Antriebskraft bzw. der Drehmomentkraft sowie davon ableitbaren Größen zu berechnen und laufend auszugeben.

7. Übungsvorrichtung nach Anspruch 6, **dadurch gekennzeichnet, dass** die Auswerteeinrichtung (40, 41) dazu eingerichtet ist, Signale von der Messanordnung (30) betreffend Zeiten der Lastwechsel zwischen den wechselweise bedienbaren Antriebselementen (16) entgegenzunehmen und anhand der von der Messanordnung (30) bestimmten Zeiten eines Lastwechsels die aufgrund der von dem Messmittel (50) gelieferten Signale berechneten Größen wechselweise einer rechten oder linken Extremität eines Trainierenden zuzuordnen.

8. Übungsvorrichtung nach Anspruch 6 oder 7, **dadurch gekennzeichnet, dass** die aufgrund der von dem Messmittel (50) gelieferten Signale berechneten Größen anhand der von der Messanordnung (30) bestimmten Zeiten eines Lastwechsels an die rechte bzw. linke Extremität ausgegeben werden.

9. Übungsvorrichtung nach einem der vorhergehenden Ansprüche, **dadurch gekennzeichnet, dass** das Schwungrad (18) eine durch Luftreibung bremsbare Vorrichtung aufweist und mit einer elektromagnetisch wirkenden Bremse (20) verbunden ist.

10. Übungsvorrichtung nach Anspruch 9, **dadurch gekennzeichnet, dass** sich die gebremste Vorrichtung in einem Gehäuse befindet, welches Mittel (15a, 15b) zur Einstellung der Menge des infolge der Bewegung des Schwungrads (18) bewegten Luftstroms aufweist.

11. Übungsvorrichtung nach Anspruch 10, **dadurch gekennzeichnet, dass** das Gehäuse Öffnungen (15a, 15b) aufweist, deren Weite und/oder Luftdurchlässigkeit verstellbar und mit deren Hilfe der durch das Gehäuse gehende Luftstrom einstellbar ist.

12. Übungsvorrichtung nach einem der Ansprüche 9 bis 11, **dadurch gekennzeichnet, dass** die durch Luftreibung bremsbare Vorrichtung ein mit dem Schwungrad (18) drehfest verbundenes Schaufelrad (21) ist.

13. Übungsvorrichtung nach Anspruch 12, **dadurch gekennzeichnet, dass** das Schaufelrad (21) eine Mehrzahl von parallel zur Drehachse ausgerichteten Schaufelflächen aufweist.

14. Verfahren zum Bestimmen der Trainingsleistung eines Trainierenden auf einer stationären ergometrischen Übungsvorrichtung nach einem der vorangehenden Ansprüche, mit den folgenden Schritten:
(i) Messen der über Antriebselemente (16) aufgebrachten Antriebskraft bzw. dieser zugeordneten Drehmomentkraft;
(ii) Detektieren der Zeiten eines Lastwechsels zwischen den wechselweise bedienbaren Antriebselementen (16); und
(iii) Berechnen und laufendes Ausgeben des zeitlichen Verlaufs der Antriebskraft bzw. der Drehmomentkraft sowie davon ableitbarer Größen aufgrund der in den Schritten (i) und (ii) erhaltenen Messwerte.

15. Verfahren nach Anspruch 14, weiterhin **gekennzeichnet durch** den folgenden Schritt:
(iv) Zuordnen der in Schritt (iii) berechneten Größen wechselweise zu einer rechten oder linken Extremität des Trainierenden in Abhängigkeit von den in Schritt (ii) detektierten Zeiten eines Lastwechsels.

16. Verfahren nach Anspruch 14 oder 15, **dadurch gekennzeichnet, dass** die Winkelgeschwindigkeit der Antriebselemente (16) aufgrund der in Schritt (ii) detektierten Zeiten eines Lastwechsels berechnet wird.

17. Verfahren nach Anspruch 16, **dadurch gekennzeichnet, dass** die Winkelgeschwindigkeit der Antriebselemente (16) verwendet wird, um die Bewegungsposition der Antriebselemente (16) zwischen einem Lastwechsel bei der Erstellung eines die Antriebskraft über den Drehwinkel der Antriebselemente (16) darstellenden Polardiagramms zu bestimmen.

## Claims

1. Stationary ergometric exercise apparatus with a hand or foot-operable drive with two alternately operable drive elements (16), preferably foot-driven pedals, wherein the drive is joined to a flywheel (18) by means of a gear mechanism, as well as with a measuring unit (50) for measuring at least one of the drive force applied via the drive and the torque related to it, and a measuring device (30) for measuring the position in motion, in particular the angular position, of the drive,
**characterised in that** the apparatus includes a wheel (19) joined to the drive so as to be synchronous in motion therewith and the measuring device (30) includes a pair of sensor devices (31,32) arranged in positions relative to the wheel (19) whereby a signal is generated when the wheel (19) is located at each of two specific angular positions, the specific angular positions being located 180° apart and corresponding to positions in motion of a load alternation between the alternately operable drive elements (16).

2. Stationary ergometric exercise apparatus according to Claim 1 wherein the measuring device (30) includes a pair of sensor pieces (32) attached to the wheel (19) and at least one sensor (31) positioned in a stationary location relative to the wheel (19), the sensor pieces (32) being movable with the wheel (19) relative to the at least one sensor (31) on operation of the drive by means of which each of the sensor pieces (32) is detected passing a sensor (31) when the wheel (19) is located at a respective one of two specific angular positions, the positions being located 180° apart and corresponding to the positions in motion of a load alternation between the alternately operable drive elements (16).

3. Stationary ergometric exercise apparatus according to Claim 1 wherein the measuring device (30) includes a pair of sensors (31) positioned in stationary locations relative to the wheel (19) and at least one sensor piece (32) attached to the wheel (19), the at least one sensor piece (32) being movable with the wheel (19) relative to the sensors (31) on operation of the drive by means of which each of the sensors (31) detects a passing sensor piece (32) when the wheel (19) is located at a respective one of two specific angular positions, the positions being located 180° apart and corresponding to the positions in motion of a load alternation between the alternately operable drive elements.

4. Stationary ergometric exercise apparatus according to Claim 2 or Claim 3 wherein the sensor pieces (32) are magnets and the sensors (31) are magnetic field sensors.

5. Stationary ergometric exercise apparatus according to any preceding claim wherein the measuring unit (50) for measuring the drive force applied via the drive includes an arm applied to a traction mechanism, in particular to a chain, of the gear mechanism, the arm pressing slightly on the side of the traction mechanism, and the measuring unit (50) further including a measuring sensor (53) to measure the restoring force applied by the traction mechanism to the arm.

6. Stationary ergometric exercise apparatus according to any preceding claim further including an evaluation device (40,41) configured to receive signals from the measuring unit (50) concerning the drive force applied and/or torque related to it, and to calculate and continuously output the temporal progress of the drive force and/or related torque, as well as variables derivable from it, on the basis of the signals delivered by the measuring unit (50).

7. Stationary ergometric exercise apparatus according to Claim 6 wherein the evaluation device (40,41) is configured to receive signals from the measuring device (30) identifying the times of load alternation between the alternately operable drive elements and, using the times of load alternation identified by the measuring device (30), to apportion the variables calculated on the basis of the signals delivered by the measuring unit (50) alternately to a right or left limb of a person in training.

8. Stationary ergometric exercise apparatus according to Claim 6 or Claim 7 wherein the variables calculated on the basis of the signals delivered by the measuring unit (50) are output on the basis of the times of load alternation identified by the measuring device (30) to the right or left limb respectively.

9. Stationary ergometric exercise apparatus according to any preceding claim wherein the flywheel (18) includes a braking device acting by air resistance and joined to an electro-magnetically-acting brake (20).

10. Stationary ergometric exercise apparatus according to Claim 9 wherein the braking device acting by air resistance is located in a housing having means (15a,15b) for adjusting the quantity of air being moved as a result of the motion of the flywheel (18).

11. Stationary ergometric exercise apparatus according to Claim 10 wherein the housing has openings (15a,15b), the size and/or air permeability of which is changeable and by means of which the flow of air passing through the housing is adjustable.

12. Stationary ergometric exercise apparatus according to any one of Claims 9 to 11 wherein the braking device acting by air resistance is a paddle wheel (21) that is joined in a rotationally locked manner to the flywheel.

13. Stationary ergometric exercise apparatus according to Claim 12 wherein the paddle wheel (21) has a plurality of paddle blades aligned parallel to the axis of rotation.

14. A method of evaluating the performance of a person in training on a stationary ergometric exercise apparatus according to any preceding claim comprising the steps of:
(i) measuring the drive force and/or torque related to it applied via the drive elements;
(ii) detecting the times of load alternation between the alternately operable drive elements; and
(iii) calculating and continuously outputting the temporal progress of the drive force and/or related torque, and variables derivable from it, on the basis of the measurements obtained in steps (i) and (ii).

15. A method according to Claim 14 further including the step of:
(iv) apportioning the variables calculated in step (iii) alternately to a right or left limb of the person in training in dependence on the times of load alternation detected in step (ii).

16. A method according to Claim 14 or Claim 15 wherein the angular velocity of the drive elements is calculated on the basis of the times of load alternation detected in step (ii).

17. A method according to Claim 16 wherein the angular velocity of the drive elements is used to determine the positions in motion of the drive elements between load alternation in the production of a polar display showing drive force against angle of rotation of the drive elements.

## Revendications

1. Dispositif d'exercice ergométrique stationnaire, comportant un système d'entraînement, actionné manuellement ou avec les pieds et muni de deux éléments d'entraînement (16) utilisables réciproquement, de préférence des pédales actionnées avec le pied, le système d'entraînement étant relié à un volant (18) par l'intermédiaire d'un engrenage, et comportant un moyen de mesure (50) pour mesurer la force motrice appliquée par l'intermédiaire du système d'entraînement ou le couple de rotation associé à celle-ci, et un système de mesure (30) pour mesurer la position dynamique, en particulier la position angulaire, du système d'entraînement,
**caractérisé en ce que** le dispositif comporte une paire de moyens de détection (31, 32) qui, par rapport à un pignon (19) relié au système d'entraînement de manière synchrone en mouvement, sont disposés en des positions par rapport au pignon (19), un signal étant produit lorsque le pignon (19) est disposé dans une parmi deux positions angulaires spécifiques, les positions angulaires spécifiques étant décalées de 180° et correspondant aux positions dynamiques d'une alternance de charge entre les éléments d'entraînement (16) utilisables réciproquement.

2. Dispositif d'exercice selon la revendication 1, **caractérisé en ce que** le système de mesure (30) comprend une paire d'éléments de détection (32) fixés sur le pignon (19) et au moins un capteur (31), le capteur (31) étant agencé de manière fixe par rapport au pignon (19), le passage d'un capteur (31) à travers chaque élément de détection (32) étant détecté au moyen des éléments de détection (32), lesquels sont mobiles avec le pignon (19) par rapport à l'au moins un capteur (31) lors du fonctionnement du système d'entraînement, lorsque le pignon (19) est agencé dans une position angulaire correspondante parmi deux positions angulaires spécifiques, les positions dynamiques étant décalées de 180° et correspondant aux positions dynamiques d'une alternance de charge entre les éléments d'entraînement (16) utilisables réciproquement.

3. Dispositif d'exercice selon la revendication 1, **caractérisé en ce que** le système de mesure (30) comprend une paire de capteurs (31) agencés de manière fixe par rapport au pignon (19) et au moins un élément de détection (32) fixé sur le pignon (19), l'au moins un élément de détection (32) étant mobile avec le pignon (19) par rapport aux capteurs (31) lors du fonctionnement du système d'entraînement, chacun des capteurs (31) détectant un élément de détection (32) passant par celui-ci, lorsque le pignon (19) se situe dans une position angulaire correspondante parmi deux positions angulaires spécifiques, les positions dynamiques étant décalées de 180° et correspondant aux positions dynamiques d'une alternance de charge entre les éléments d'entraînement (16) utilisables réciproquement.

4. Dispositif d'exercice selon la revendication 2 ou 3, **caractérisé en ce que** les éléments de détection (32) sont des aimants et les capteurs (31) sont des capteurs à champ magnétique.

5. Dispositif d'exercice selon l'une quelconque des revendications précédentes, **caractérisé en ce que** le moyen de mesure (50), destiné à mesurer la force motrice, est un bras, qui est en appui contre un moyen de traction, en particulier une chaîne, et qui enfonce faiblement le moyen de traction, et **en ce que** le moyen de mesure (50) comporte en outre un capteur de mesure (53) destiné à mesurer la force de commande exercée par le moyen de traction sur le bras.

6. Dispositif d'exercice selon l'une quelconque des revendications précédentes, **caractérisé par** un dispositif d'analyse (40, 41), qui est conçu pour recevoir des signaux du moyen de mesure (50) concernant la force motrice appliquée ou le couple de rotation associé à celle-ci, et pour calculer et pour éditer en continu, à l'appui des signaux délivrés par le moyen de mesure (50), l'évolution dans le temps de la force motrice ou du couple de rotation, ainsi que les grandeurs pouvant être dérivées de celle-ci.

7. Dispositif d'exercice selon la revendication 6, **caractérisé en ce que** le dispositif d'analyse (40, 41) est conçu en outre pour recevoir des signaux délivrés par le système de mesure (30) et relatifs aux durées de l'alternance de charge entre les éléments d'entraînement (16) utilisables réciproquement, et pour associer les grandeurs, calculées à l'appui des signaux délivrés par le moyen de mesure (50), en alternance d'une extrémité droite ou gauche de la personne effectuant l'exercice, en fonction des durées d'une alternance de charge définies par le système de mesure (30).

8. Dispositif d'exercice selon la revendication 6 ou 7, **caractérisé en ce que** les grandeurs calculées à l'appui des signaux délivrés par le moyen de mesure (50) en fonction des durées d'une alternance de charge définies par le système de mesure (30) sont éditées respectivement sur la jambe droite et la jambe gauche.

9. Dispositif d'exercice selon l'une quelconque des revendications précédentes, **caractérisé en ce que** le volant (18) comporte un dispositif apte à être freiné par le frottement de l'air et est relié à un frein (20) agissant par voie électromagnétique.

10. Dispositif d'exercice selon la revendication 9, **caractérisé en ce que** le dispositif freiné est contenu dans un carter qui comporte des moyens (15a, 15b) pour le réglage de la quantité de flux d'air mise en mouvement par suite du mouvement du volant (18).

11. Dispositif d'exercice selon la revendication 10, **caractérisé en ce que** le carter comporte des ouvertures (15a, 15b) dont la largeur et/ou la pénétrabilité de l'air sont réglables et au moyen desquelles il est possible de régler le flux d'air traversant le carter.

12. Dispositif d'exercice selon l'une quelconque des revendications 9 à 11, **caractérisé en ce que** le dispositif apte à être freiné par le frottement de l'air est une roue à palettes (21) reliée de manière solidaire en rotation avec le volant (18).

13. Dispositif d'exercice selon la revendication 12, **caractérisé en ce que** la roue à palettes (21) comporte une pluralité de surfaces de palettes orientées parallèlement à l'axe de rotation.

14. Procédé permettant de déterminer la puissance d'exercice d'une personne effectuant l'exercice sur un dispositif d'exercice ergométrique stationnaire selon l'une quelconque des revendications précédentes, le procédé comprenant les étapes suivantes consistant à :
(i) mesurer la force motrice appliquée par des éléments d'entraînement (16) ou le couple de rotation associé à celle-ci ;
(ii) détecter les durées d'une alternance de charge entre les éléments d'entraînement (16) utilisables réciproquement ; et
(iii) calculer et éditer en continu la variation dans le temps de la force motrice ou du couple de rotation ainsi que les grandeurs pouvant en dériver à l'appui des valeurs de mesure obtenues aux étapes (i) et (ii).

15. Procédé selon la revendication 14, **caractérisé par** l'étape suivante consistant à :
(iv) associer les grandeurs calculées à l'étape (iii) en alternance à une extrémité droite ou gauche de la personne effectuant l'exercice en fonction des durées d'une alternance de charge détectées à l'étape (ii).

16. Procédé selon la revendication 14 ou 15, **caractérisé en ce que** la vitesse angulaire des éléments d'entraînement (16) est calculée à l'appui des durées d'une alternance de charge détectées à l'étape (ii).

17. Procédé selon la revendication 16, **caractérisé en ce que** la vitesse angulaire des éléments d'entraînement (16) est utilisée pour déterminer la position dynamique des éléments d'entraînement (16) entre une alternance de charge lors de l'établissement d'un diagramme polaire représentant la force motrice sur l'angle de rotation des éléments d'entraînement (16).
